# FASCICULE DE BREVET EUROPEEN

(11) **EP 1 603 609 B1**
(45) Date de publication et mention de la délivrance du brevet: **18.05.2011**
(21) Numéro de dépôt: 04720673.5
(22) Date de dépôt: 15.03.2004
(51) Int. Cl.: A61M 1/34

(54) **DISPOSITIF DE TRAITEMENT DE SANG AVEC EXTRACTION SELECTIVE DE SOLUTES**
VORRICHTUNG ZUR BLUTBEHANDLUNG MIT SELEKTIVER ENTFERNUNG GELÖSTER SUBSTANZEN
BLOOD TREATMENT DEVICE WITH SELECTIVE SOLUTE EXTRACTION

(30) Priorité: 17.03.2003 FR 0303257
(43) Date de publication de la demande: 14.12.2005
(60) Demande divisionnaire: 10016027.4
(73) Titulaire: Gambro Lundia AB, 22 643 Lund (SE)
(72) Inventeur: CHEVALLET, Jacques, F-69360 Serezin du Rhone (FR); MERCIER, Guy, F-69500 Bron (FR); RADA, Hiram, F-69008 Lyon (FR); MONCHI, Mehran, F-77190 Dammarie Les Lys (FR)
(74) Mandataire: Ponzellini, Gianmarco
(86) Numéro de dépôt international: PCT/IB2004/000896
(87) Numéro de publication internationale: WO 2004/082733

(56) Documents cités:
- WO-A-95/04560
- WO-A-96/28198
- DE-A- 4 102 693
- DE-A- 19 854 338
- US-A- 4 243 532
- US-A- 5 108 612

## Description

### Domaine de l'invention:

L'objet de la présente demande est la filtration de sang afin de séparer et extraire sélectivement des molécules de taille choisie par le biais de systèmes extracorporels pour la séparation de substances.

De tels systèmes sont utilisés pour le traitement de sang comportant des solutés de masse moléculaire différente. De telles substances sont à titre d'exemple l'urée d'une masse moléculaire de 60 daltons, le phosphate (96-97 daltons), la créatinine (113 daltons), la vitamine B₁₂ (1355 daltons), l'inuline (5200 daltons), la béta 2-microglobuline (12000 daltons), l'albumine (58000 daltons).

On appellera par la suite molécules de petite taille les molécules dont la masse moléculaire est inférieure à environ 2000 daltons, molécules de masse moyenne les molécules dont la masse moléculaire est comprise entre 2000 et 50000 daltons et molécules de grande masse les molécules dont la masse moléculaire est supérieure à 50000 daltons (les protéines par exemple).

### Etat de la technique:

De tels systèmes sont souvent des systèmes à membrane extracorporelle pour la séparation de solutés de masse inférieure à l'albumine appliqués à des traitements d'insuffisance rénale.

Des progrès ont toujours été recherchés notamment pour améliorer la clairance, pour diminuer la durée de traitement

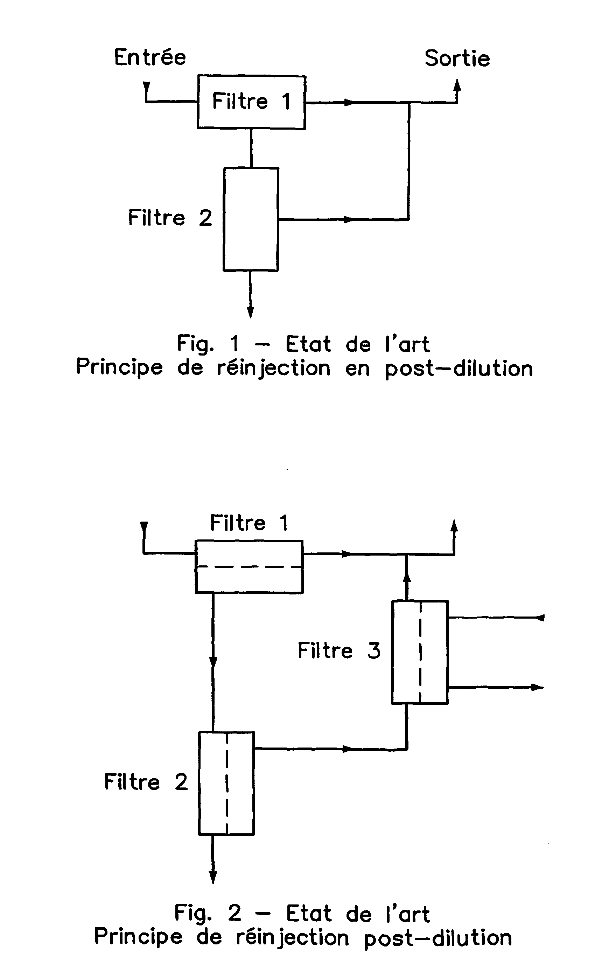

It is known from WO96/28198 a two-stage size séparation circuit based on the available filtration technology and dialysis hardware for the treatment of acute renal failure, which is said to be capable of offering high single-pass elimination of the free virus (and boasting a time constant of approx. 10 to 15 minutes for the systemic clearance): such a circuit shows a closed recirculation sub-circuit (see for example figure 2).

At the same time, the circuit depicted in WO96/28198 shows a line, referenced by number 17, which is an inlet line (allowing the liquid to enter into the treatment unit 13): therefore, such a line is not a "discharge line" of the treatment unit.

et pour rendre de tels systèmes plus simples et moins chers. On rappelle que la clairance d'un soluté est le taux de soluté dans le sang nettoyé pour un volume donné.

Dans le domaine de la dialyse, les premières membranes utilisées étaient hautement perméables à des petits solutés ayant une taille jusqu'à 200 dalton. La clairance des petits solutés dépend de la perméabilité et la capacité de diffusion de la membrane utilisée.

Le manque de perméabilité des premières membranes pour certains solutés à taille moléculaire moyenne dans la gamme des vitamines B₁₂ (1355 daltons) a été tenu responsable de l'apparition de neuropathies multiples urémiques.

Concernant l'amélioration de la clairance de moyennes molécules, une première solution était d'ajouter au flux diffusif à travers la membrane un flux convectif en utilisant des membranes à flux élevé ayant un point de coupure (« cut off» en anglais) de taille moléculaire de 40000 dalton. On rappelle que le point de cut off d'une membrane est défini par la taille moléculaire à laquelle seulement 10% des solutés passent à travers la membrane.

Mais les problèmes rencontrés lors de la mise en oeuvre de cette solution sont la difficulté de contrôler le taux d'ultrafiltration obtenu par le flux convectif, et la perte élevée de composants utiles du plasma tels que des hormones, des vitamines et des acides aminés.

Une deuxième solution concernant l'amélioration de la clairance de moyennes molécules fut alors l'hémofiltration, c'est-à-dire un procédé purement convectif d'élimination de solutés par la membrane. Mais un tel procédé extrait beaucoup de liquide et nécessite en compensation une dilution de liquide stérile en pré-dilution et / ou en post-dilution et l'utilisation d'une membrane hautement perméable aux solutés de taille moléculaire atteignant 40 000 dalton. Seulement, en mode purement convectif la clairance dépend du mode de dilution (pré ou post-dilution), du débit du sang et du débit de l'infusat. Avec une hémofiltration conventionnelle, la clairance des petites molécules est inférieure à celle obtenue en mode hémodialyse. Cette clairance en mode hémofiltration pourrait atteindre celle de l'hémodialyse si l'on augmente le débit de l'infusat, le débit sanguin et la surface de membrane. Mais tout ceci n'est pas pratique, augmente le coût du traitement et provoque la perte d'acides aminés et d'hormones. De plus, le débit sanguin est limité, notamment pour les patients ayant un accès sanguin faible.

Concernant la clairance des petites molécules, quand il a été constaté cette clairance en mode hémofiltration était limitée, les deux phénomènes d'hémofiltration et d'hémodialyse ont été combinés. Cette technique simultanée pour une même membrane est connue sous le nom d'hémodiafiltration. Mais les problèmes constatés sont la difficulté du contrôle précis du flux d'hémofiltration, la perte élevée d'hormones et d'acides aminés, la complexité du système, les grandes quantités de liquide stérile et de dialysat nécessaires et par conséquent le coût d'un tel traitement.

Ainsi, l'utilisation d'un seul filtre travaillant en modes opératoires différents ne résolvait toujours pas notamment les problèmes de pertes de molécules d'une certaine taille et de coût du traitement.

Une proposition a alors été faite par les docteurs J.C. Kingswood et F.D.Thompson. Elle avait pour objet une hémofiltration continue sans liquide de ré-injection: le traitement de l'ultrafiltrat était assuré par une deuxième membrane fonctionnant également en ultrafiltration spontanée. La figure 1 représente le dispositif de dialyse issu de cette proposition.

Il s'agit de traiter un premier ultrafiltrat issu d'une première membrane à fibres creuses par le passage à travers une seconde membrane à fibres creuses en mode ultrafiltration. Une première ultrafiltration est effectuée à travers une première membrane à flux élevé et non perméable aux molécules de masse moléculaire supérieure à 10000 dalton. La taille des orifices à travers la seconde membrane est plus petite que celle de la première.

Comme indiqué sur la figure 1, en sortie de la première membrane, le liquide non filtré contenant principalement des molécules de grande masse est amené en ré-injection au patient. Le premier ultrafiltrat contenant des molécules de petite et moyenne masse est filtré à travers la seconde membrane. Le liquide non filtré par la seconde membrane contenant principalement des molécules de masse moyenne est recueilli dans une poche de déchet. Le deuxième ultrafiltrat contenant principalement des molécules de petite masse est réinjecté en post-dilution sur la ligne veineuse du patient.

Ceci permet de ne pas consommer trop de liquide stérile en post-injection, et de réinjecter au patient un liquide contenant peu de particules de taille moyenne.

Mais une perte élevée de nutriments, d'acides aminés, de glucose et de vitamines a été constatée; et la clairance des petits ions comme le potassium n'est pas bonne.

Aussi, un autre dispositif a été réalisé en dialyse. Il a été considéré que les molécules urémiques à éliminer avaient une masse moléculaire inférieure à 200 daltons et une masse moléculaire comprise entre 10000 et 40000 daltons.

Cette considération fut à l'origine de la réalisation d'un dispositif comportant trois filtres, représenté en figure 2. Un premier filtre possède un point de cut off d'environ 40000 daltons. Le sang passe à travers ce premier filtre et produit en sortie un premier filtrat contenant petites et moyennes molécules, c'est-à-dire des molécules de masse moléculaire inférieure à 40000 daltons. Les solutés de masse comprise entre 10000 et 40000 daltons sont ensuite éliminés par ultrafiltration à travers le filtre 2 ayant un point de cut off inférieur à 10000 daltons. Puis le deuxième filtrat est traité en hémofiltration avec une membrane ayant un point de coupure d'environ 200 daltons. Ainsi, le filtrat purifié contenant les solutés entre 200 et 10000 daltons est retourné en post infusion vers le patient qui reçoit aussi les molécules de masse supérieure à 40000 daltons.

Mais la clairance de tous les solutés dépend du taux d'ultrafiltration dans le filtre 1, qui ne peut pas être supérieure à 30% du flux sanguin, ce qui est une valeur faible en comparaison avec l'hémodialyse conventionnelle. Aussi le coût est élevé.

Enfin, le brevet US 6,193,681 décrit un appareil pour traiter la septicémie dans le sang représenté en figure 3. Le sang passe par un dispositif d'irradiation à utraviolet puis à travers un hémoconcentrateur avant d'être ré-injecté dans le patient. Un circuit secondaire est branché à une deuxième sortie de l'hémoconcentrateur dont le fluide sortant passe à travers un filtre, puis un module de membrane et une source de dilution avant d'être injecté en amont de l'hémoconcentrateur.

Il existe par ailleurs un problème équivalent pour la plasmaphérèse. La plasmaphérèse d'échange thérapeutique est pratiquée sur un patient dont le plasma contient une ou plusieurs substances nocives ou toxiques.

L'élimination des solutés du plasma s'effectue selon le même principe que l'élimination des solutés du sang, une différence étant la plus grande masse moléculaire des solutés à extraire du plasma.

Ainsi, des problèmes récurrents ont été rencontrés lors de la réalisation des dispositifs de l'art antérieur:

La consommation élevée du liquide de perfusion,

La perte élevée de nutriments, d'acides aminés, de glucose et de vitamines,

La clairance faible des solutés,

Le coût du dispositif comportant plusieurs filtres et pompes.

Le problème posé par la demande est d'éliminer sélectivement des molécules par gamme(s) de masse moléculaire avec une bonne clairance et de consommer très peu de liquide stérile. Par exemple, pour des patients en état de septicémie, on souhaite éliminer beaucoup de molécules de taille moyenne tout en gardant une élimination correcte de molécules de petite masse. On rappelle que la septicémie est caractérisée par des décharges importantes et répétées de germes pathogènes figurés à partir d'un foyer primitif.

Un problème auxiliaire serait l'adaptation optimale d'un tel système à une thérapie de longue durée pratiquée en soins intensifs sans risque d'obturation des filtres. L'adaptation s'effectuerait par le choix des modes de fonctionnement des différents filtres, de l'utilisation et du placement opportun de moyens de réglage de débit, des débits commandés, et l'architecture hydraulique des lignes.

### Exposé de l'invention:

Pour atteindre le problème posé on prévoit, conformément à l'invention, un dispositif de traitement extracorporel de sang tel que revendiqué.

Il est aussi décrit, mais ne fait pas de l'invention, un procédé de traitement extracorporel de sang pour la mise en oeuvre sur un dispositif de traitement extracorporel de sang comportant un échangeur 1 sur lequel sont branchées une ligne d'entrée 10 du sang et une ligne de sortie 11 du sang, et une unité de traitement 21, le procédé comprenant les étapes suivantes: envoyer le sang sur la ligne d'entrée 10 reliée à 1' échangeur 1, effectuer une première filtration du sang via l'échangeur 1 en produisant un premier filtrat, effectuer au moins une deuxième filtration du premier filtrat via l'unité de traitement 21 en produisant un deuxième filtrat, renvoyer le deuxième filtrat sur la ligne d'entrée 10 pour effectuer une pré-dilution du sang à traiter, envoyer le sang en sortie de l'échangeur 1 vers la ligne de sortie 11.

D'autres caractéristiques de l'invention apparaîtront à la lecture de la description qui suit.

### Brève description des dessins:

On se reportera aux dessins annexés sur lesquels:
la figure 1 représente l'état de l'art concernant l'utilisation de deux filtres avec points de coupure différents et avec une ré-injection en post-dilution;
la figure 2 représente l'état de l'art concernant l'utilisation de trois filtres avec points de coupure différents et avec une ré-injection en post-dilution;
la figure 3 représente l'état de l'art du brevet US6,193,681;
les figures 4 à 9 sont des représentations schématiques du dispositif de traitement de liquide physiologique selon l'invention, ainsi que des variantes de réalisation;
les figures 10 et 11 représentent les résultats estimés en terme de clairance en fonction de la taille moléculaire des solutés pour deux configurations de dispositif selon l'invention.

### Exposé détaillé de l'invention:

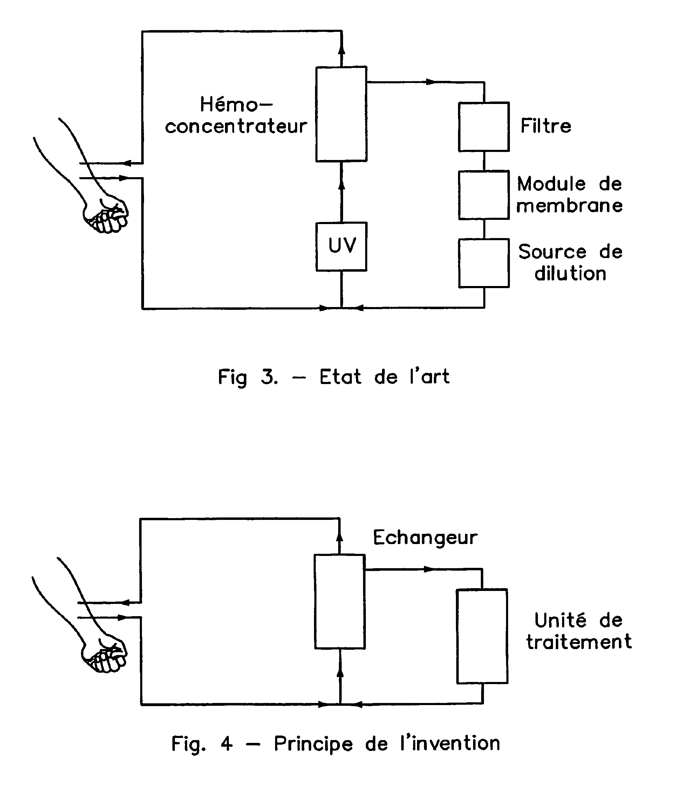

La figure 4 représente de façon schématisée le principe de l'invention : le passage du sang dans une ligne d'entrée, son entrée dans un échangeur et sa sortie de l'échangeur vers une ligne de sortie, ainsi que le traitement du premier filtrat par une unité de traitement et l'injection du liquide en sortie de l'unité de traitement en pré-dilution sur la ligne artérielle. On peut voir ce concept comme une « cascade » de filtration avec injection du filtrat final en pré-dilution du circuit : un premier filtrat est une deuxième fois filtré, et le deuxième filtrat est injecté en entrée du premier filtre, ou en « pré-dilution ».

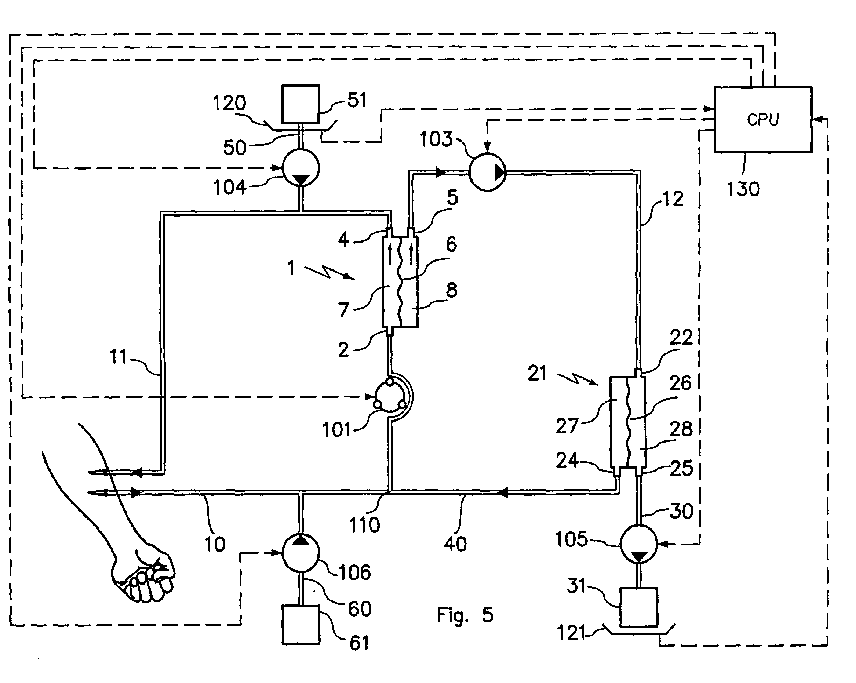

La figure 5 représente le dispositif de traitement extracorporel de sang de l'invention comprenant un échangeur 1 comportant une première entrée 2 pour le sang à traiter, une première sortie 4 de fluide et une deuxième sortie 5 de fluide, une ligne d'entrée 10 du sang à traiter ou ligne artérielle raccordée à la première entrée 2 de l'échangeur 1, une ligne de sortie du sang ou ligne veineuse 11 raccordée à la première sortie 4 de l'échangeur 1. Une unité de traitement 21 comporte une première entrée 22 de fluide et une première sortie 24 de fluide, la deuxième sortie 25 de l'échangeur 1 est en communication de fluide avec la première entrée 22 de l'unité de traitement 21, et la première sortie 24 de l'unité de traitement 21 est en communication de fluide avec la ligne d'entrée 10.

La communication de fluide entre la première entrée 22 de l'unité de traitement 21 et la deuxième sortie 5 de l'échangeur 1 est effectuée par une canalisation 12.

L'échangeur 1 peut comporter une membrane semi-perméable 6 le divisant en une première chambre 7 et une deuxième chambre 8. La première entrée 2 de l'échangeur est en communication de fluide avec la première chambre 7 de l'échangeur et la première sortie 4 de l'échangeur est en communication de fluide avec la première chambre 7 de l'échangeur et la deuxième sortie 5 de l'échangeur est en communication de fluide avec la deuxième chambre 8 de l'échangeur.

La ligne d'entrée 10 du sang dite « ligne artérielle » raccordée à la première entrée 2 de l'échangeur 1, la ligne de sortie 11 du sang dite « ligne veineuse » raccordée à la première sortie 4 de l'échangeur et la première chambre 7 de l'échangeur font partie d'un circuit extracorporel de traitement de sang.

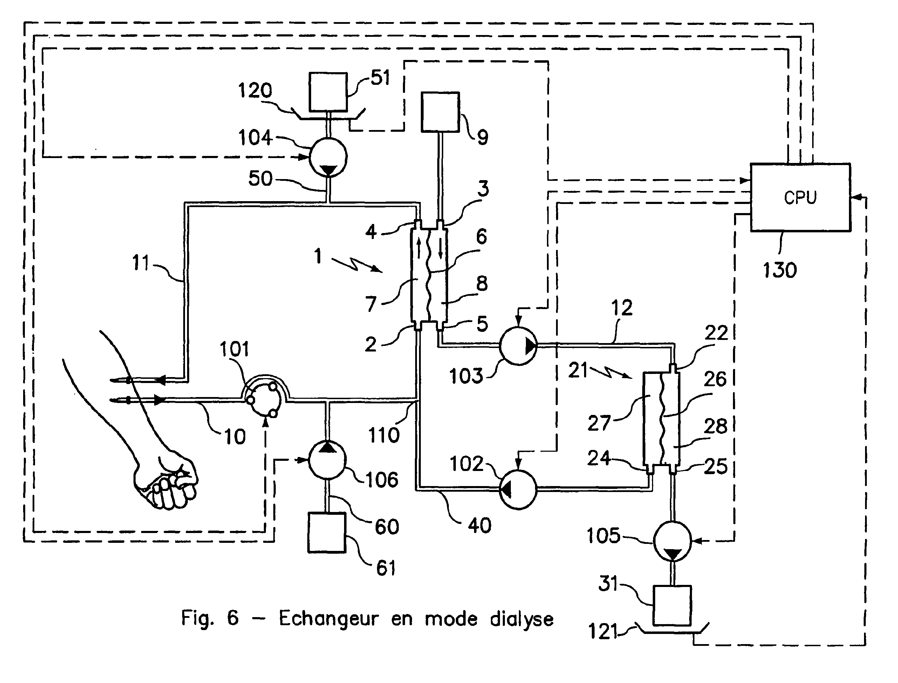

Dans un mode de réalisation représenté en figure 6, l'échangeur 1 peut comporter une deuxième entrée 3 en communication de fluide avec la deuxième chambre 8 et en communication de fluide avec une première source de liquide de dialyse 9. Dans ce mode de fonctionnement, le sang et le liquide de dialyse circulent en sens inverse dans chacune des deux chambres.

La figure 5 représente l'unité de traitement 21 avec une membrane semi-perméable 26 la divisant en une première chambre 27 et une deuxième chambre 28.

Aussi, l'unité de traitement 21 peut comporter une deuxième sortie de fluide 25.

Aussi, la première sortie 24 de l'unité de traitement 21 est en communication de fluide avec la première chambre 27 de l'unité de traitement 21 et la deuxième sortie 25 de l'unité de traitement 21 est en communication de fluide avec la deuxième chambre 28 de l'unité de traitement 21.

De manière alternative, la première entrée 22 de l'unité de traitement 21 peut être en communication de fluide soit avec la deuxième chambre 28 de l'unité de traitement 21 soit avec la première chambre 27 de l'unité de traitement 21.

La deuxième sortie 25 de l'unité de traitement 21 est en communication de fluide avec une première ligne de décharge 30 de liquide usé, ladite première ligne de décharge 30 pouvant relier la deuxième sortie 25 de l'unité de traitement 21 à un égout ou à un premier réservoir de liquide usé 31.

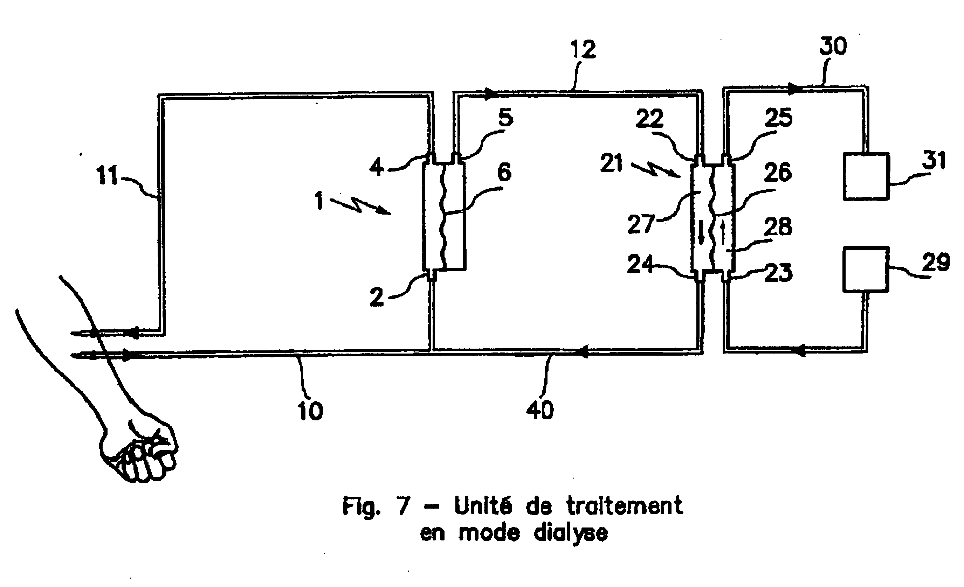

L'unité de traitement 21 peut également comporter une deuxième entrée 23, la deuxième entrée 23 étant en communication de fluide avec la deuxième chambre 28 et avec une deuxième source de liquide de dialyse 29. Dans ce mode de fonctionnement de l'unité de traitement représenté en figure 7, le liquide de dialyse circule en contresens par rapport au liquide physiologique arrivant par la première entrée 22.

L'échangeur 1 et l'unité de traitement 21 ont des caractéristiques différentes. En effet, la membrane 6 de l'échangeur 1 peut être une membrane à flux élevé et la membrane 26 de l'unité de traitement 21 peut être une membrane à flux faible.

Une membrane à flux faible a une perméabilité à l'eau faible. Le coefficient d'ultrafiltration est approximativement entre 2 et 10mL/h,mmHg,m². Une membrane à flux élevé a une perméabilité à l'eau plus élevée. Le coefficient d'ultrafiltration est approximativement entre 20 et 50mL /h,mmHg,m².

L'échangeur ou l'unité de traitement peut comporter une membrane à fibres creuses (on parle également de filtre capillaire) ou une membrane à fibres planes, c'est-à-dire à plaques.

Aussi, la perméabilité aux molécules de la membrane 6 de l'échangeur 1 est supérieure à la perméabilité aux molécules de la membrane 26 de l'unité de traitement 21 au moins au-dessus d'une certaine masse moléculaire.

Plus particulièrement, on peut définir un rapport ou une différence entre les points de coupure de la première membrane et de la deuxième membrane. Ainsi, on peut considérer que le rapport du point de coupure de la première membrane sur le point de coupure de la deuxième membrane est inférieur ou égal à 3. D'une autre manière, on peut considérer que la différence de point de coupure entre la première membrane et la deuxième membrane est comprise entre 20000 et 30000 daltons. On peut envisager que le point de coupure de la première membrane soit inférieur ou égal à 40000 daltons, que le point de coupure de la deuxième membrane soit inférieur ou égal à 10000 daltons. Dans un mode utilisé le point de coupure de la première membrane est approximativement égal à 40 000 daltons et le point de coupure de la deuxième membrane est approximativement égal à 10 000 daltons.

Afin de ré-infuser de l'eau au patient en traitement, on peut brancher sur la ligne de sortie 11 une ligne de post-dilution 50 reliée à une première source de liquide stérile 51 et/ou sur la ligne d'entrée 10 une ligne de pré-dilution 60 reliée à une deuxième source de liquide stérile 61.

Une canalisation 40 met en communication de fluide la première sortie 24 de l'unité de traitement 21 et la première entrée 2 de l'échangeur 1.

La ligne de pré-dilution 60 peut être branchée directement sur ladite canalisation 40 ou directement sur la ligne d'entrée 10.

Les différentes sources de liquide stérile 51, 61 peuvent être des poches de liquide stérile et/ou peuvent être obtenues par une préparation en ligne de liquide stérile à partir de l'eau du réseau hydrique.

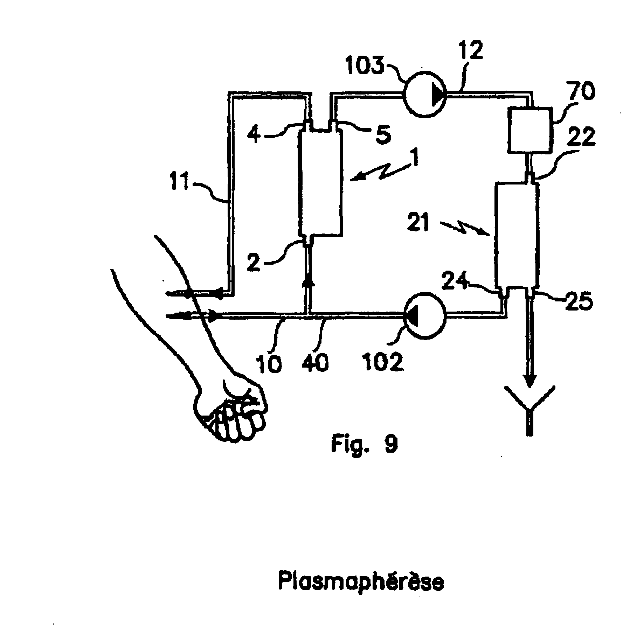

Dans l'application de la présente invention au cas particulier de la plasmaphérèse, représenté en figure 9 , l'échangeur est un plasma-filtre. Le plasma-filtre a un point de coupure compris entre un million et cinq million daltons.

Là encore, l'échangeur ou l'unité de traitement peut comporter une membrane à fibres creuses (on parle également de filtre capillaire) ou une membrane à fibres planes, c'est-à-dire à plaques.

Aussi, l'unité de traitement 21 comprend une unité capable de fixer au moins une substance donnée. Elle peut être une cartouche d'adsorption, un réacteur, par exemple une cellule électrophérèse.

L'unité de traitement peut comporter une membrane semi-perméable 26 la divisant en une première chambre 27 comportant une première sortie 24 et une deuxième chambre 28 comportant une première entrée 22 et une deuxième sortie 25. La deuxième sortie est reliée à une ligne de décharge. L'unité de traitement peut avoir un point de coupure inférieur ou égal à 250000 daltons.

Le point de coupure pourra être inférieur ou égal à 200000 daltons.

L'unité de traitement peut avoir un point de coupure tel que la membrane laisse passer 100% des molécules d'albumine à 58000 daltons.

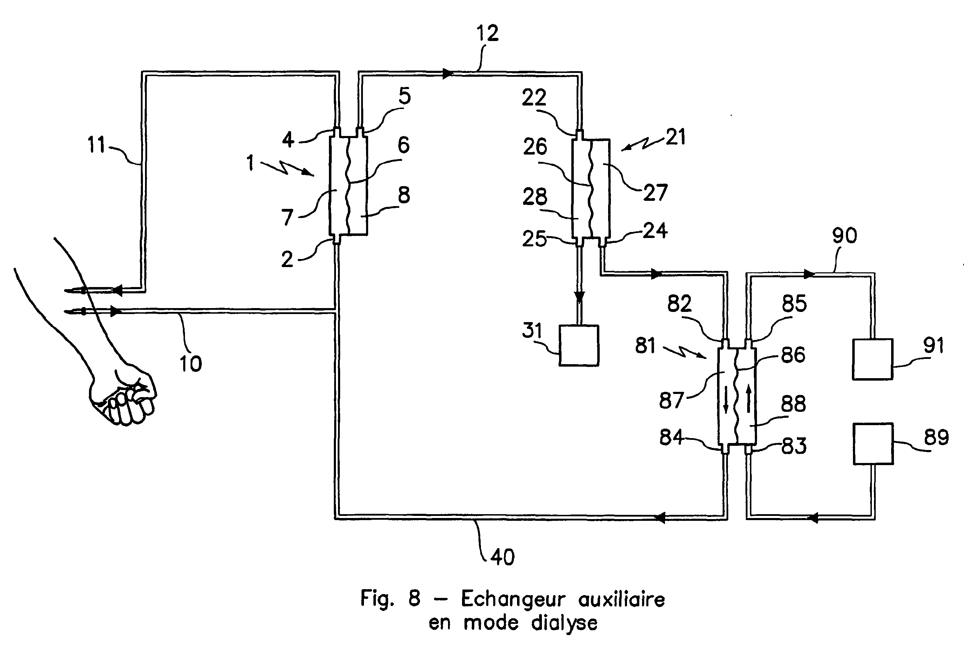

Un autre aspect de l'invention est de rajouter un troisième moyen de filtration pour effectuer une élimination supplémentaire de gamme de masse moléculaire, représenté en figure 8. Le dispositif peut comporter au moins un échangeur auxiliaire 81 ayant une membrane 86 le séparant en une première chambre 87 en communication de fluide avec une première entrée 82 et une première sortie 84 et en une deuxième chambre 88 en communication de fluide avec au moins une deuxième sortie 85. Le point de coupure d'un tel échangeur auxiliaire serait inférieur aux points de coupure des deux autres membranes (6, 26).

La première entrée 82 de l'échangeur auxiliaire 81 est en communication de fluide avec la deuxième sortie 24 de l'unité de traitement 21 et une des deux sorties 84, 85 de l'échangeur auxiliaire 81 est en communication de fluide avec la première entrée 2 de l'échangeur 1.

Une deuxième ligne de décharge 90 de liquide usé relie l'autre sortie 84, 85 de l'échangeur auxiliaire 81 à un égout, l'égout pouvant être un deuxième réservoir de liquide usé 91.

La figure 8 représente l'échangeur auxiliaire fonctionnant en mode dialyse : l'échangeur auxiliaire 81 comporte une deuxième entrée 83 en communication de fluide avec la deuxième chambre 88 de l'échangeur auxiliaire 81 et en communication de fluide avec une troisième source de liquide de dialyse 89, la première sortie 84 de l'échangeur auxiliaire 81 étant en communication de fluide avec la première entrée 82 de l'échangeur 1, la deuxième sortie 85 de l'échangeur auxiliaire 81 étant en communication de fluide avec un égout 91 par une deuxième ligne de décharge de liquide usé 90.

Le choix des trois membranes sera effectué très précisément en fonction du patient et du traitement à adopter concernant la masse des molécules que l'on souhaite éliminer ou garder. La première membrane 6 permet de travailler sur les molécules de grande masse moléculaire (de façon préférée en mode hémofiltration), la deuxième membrane 26 permet de travailler sur les molécules de moyenne masse moléculaire (de façon préférée en mode hémofiltration), et la troisième membrane 86 permet de travailler sur les molécules de petite masse moléculaire donc de façon préférée en mode dialyse. Ceci n'empêche pas l'échangeur auxiliaire 81 de fonctionner en mode ultrafiltration également. Le choix du fonctionnement permet de personnaliser le traitement et d'accéder à un fonctionnement optimal des membranes sans trop d'obturation.

Concernant le réglage des différents débits de fluide, il est prévu des premiers moyens de réglage de débit 101 de liquide actifs sur la ligne d'entrée 10 reliée à la première entrée 2 de l'échangeur 1.

Alternativement, les premiers moyens de réglage de débit 101 peuvent être exactement entre la première entrée 2 de l'échangeur 1 et le point de branchement 110 reliant la ligne d'entrée à la canalisation ou en amont du point de branchement 110 reliant la ligne d'entrée 10 à la canalisation 40.

Dans la première alternative, la dépression de la canalisation 40 nécessite une pression positive plus faible sur la conduite 12 pour atteindre la pression trans-membranaire (TMP) de la membrane 26 souhaitée.

Aussi, dans la première alternative, il n'est pas nécessaire d'avoir une pompe sur la canalisation 40 : une seule pompe 101 suffit pour la canalisation 40 et la ligne artérielle 11.

Dans la deuxième alternative, des deuxièmes moyens de réglage de débit 102 de liquide actifs sont sur la deuxième canalisation 40 reliant la première sortie 24 de l'unité de traitement 21 à la première entrée 2 de l'échangeur 1.

Il est également prévu des troisièmes moyens de réglage de débit 103 de liquide actifs sur la première conduite 12 reliant la deuxième sortie 5 de l'échangeur 1 à une des entrées 22, 23 de l'unité de traitement 21.

Aussi des quatrièmes moyens de réglage de débit 104 de liquide actifs peuvent être branchés sur la ligne de post-dilution 50.

Des cinquièmes moyens de réglage de débit de liquide 105 actifs peuvent être branchés sur la ligne de décharge 30 de liquide usé reliant la deuxième sortie 25 de l'unité de traitement 21 à un égout 31.

Dans la configuration ayant au moins les trois moyens de réglage de débit 101 sur la ligne d'entrée, 102 sur la deuxième canalisation 40 et 105 sur la ligne de décharge 30, il faudra veiller tout particulièrement aux différents débits imposés et compatibles.

Des sixièmes moyens de réglage de débit de liquide 106 actifs peuvent être branchés sur la ligne de pré-dilution 60.

Ces moyens de réglage de débit 101, 102, 103, 104 et 105 peuvent être des pompes et/ou des vannes. En particulier les moyens de réglage de débit sur la ligne de décharge 30, ou sur la ligne 50 de post-dilution ou la ligne 60 de pré-dilution seront des vannes.

Dans un mode de réalisation particulier, la première source 51 de liquide stérile pour la post-dilution est une poche de liquide stérile et le premier réservoir 31 de liquide usé relié à la ligne de décharge en sortie de l'unité de traitement est une poche de liquide usé. Le dispositif comporte une première balance 120 pour mesurer le poids de la poche de liquide stérile 51 et une deuxième balance 121 pour mesurer le poids de la poche de liquide usé 31. Alternativement, une unique balance 120, 121 peut mesurer le poids total de la poche de liquide stérile 51 et de la poche de liquide usé 31.

Dès lors, une unité de calcul et de commande 130 va recevoir les signaux émis par au moins une balance 120, 121 et contrôler les moyens de réglage de débit de liquide 101, 102, 103, 104, 105.

L'unité de calcul et de commande calcule périodiquement le débit réel ou un paramètre fonction du débit réel par exemple à partir du poids et de l'intervalle de temps entre chaque deux mesures. Elle comparera le débit réel mesuré au débit souhaité et sera en mesure de contrôler un ou plusieurs moyens de réglage de débit de liquide (101, 102, 103, 104, 105).

Ainsi, les quantités de liquide stérile et de liquide usé, ou leur différence peuvent être connues et contrôlées pendant le traitement. Connaissant lesdits poids, l'unité de commande et de contrôle pourra obtenir une quantité souhaitée de solution liquide stérile et de liquide usé. L'équilibre hydrique pourra être bien commandé.

Le dispositif décrit précédemment est applicable à la plasmaphérèse.

Il est aussi décrit, mais ne fait pas de l'invention, un procédé de traitement extracorporel de sang pour la mise en oeuvre sur un dispositif de traitement extracorporel de sang comportant un échangeur 1 sur lequel sont branchées une ligne d'entrée 10 du sang et une ligne de sortie 11 du sang, et une unité de traitement 21, le procédé comprenant les étapes suivantes : envoyer le sang sur la ligne d'entrée 10 reliée à la première entrée de l'échangeur 1, effectuer une première filtration du sang via l'échangeur 1 en produisant un premier filtrat sortant par la deuxième sortie de l'échangeur,
effectuer au moins une deuxième filtration du premier filtrat via l'unité de traitement 21 en produisant un deuxième filtrat,
renvoyer le deuxième filtrat sortant de la première sortie de l'unité de traitement sur la ligne d'entrée 10 pour effectuer une pré-dilution du sang à traiter,
envoyer le sang à travers la première sortie de l'échangeur vers la ligne de sortie 11.

Particulièrement, le procédé a une deuxième filtration effectuée à travers une membrane semi-perméable 26 dans une unité de traitement 21 divisée en une première chambre 27 et une deuxième chambre 28 produisant en sortie d'une part le deuxième filtrat et envoyant d'autre part en sortie le liquide non filtré vers une ligne d'égout 30.

Une autre caractéristique du procédé est que la première filtration est effectuée à travers une membrane semi-perméable 6 divisant de l'échangeur 1 en une première chambre 7 et une deuxième chambre 8.

Une autre caractéristique du procédé est que la membrane 26 de l'unité de traitement filtre des molécules de masse moléculaire inférieure à la masse moléculaire des molécules filtrées de la membrane 16 de l'échangeur.

Le procédé comprent l'étape de perfuser un liquide stérile dans la ligne de sortie 11 du sang de l'échangeur.

Le procédé comprend l'étape de perfuser un liquide stérile dans la ligne d'entrée 10 du sang de l'échangeur.

Le procédé emploie une membrane 16 de l'échangeur ayant un point de coupure inférieur à 40000 daltons.

Le procédé emploie une membrane 16 de l'unité de traitement ayant un point de coupure inférieur à 10000 daltons.

Le traitement effectué est une plasmaphérèse et l'unité de traitement fixe au moins une certaine substance donnée.

Selon une autre caractéristique de l'invention, la membrane 16 de l'échangeur a un point de coupure compris entre un million et cinq millions daltons.

Selon une autre caractéristique de l'invention, la membrane 16 de l'unité de traitement a un point de coupure inférieur à 250000 daltons.

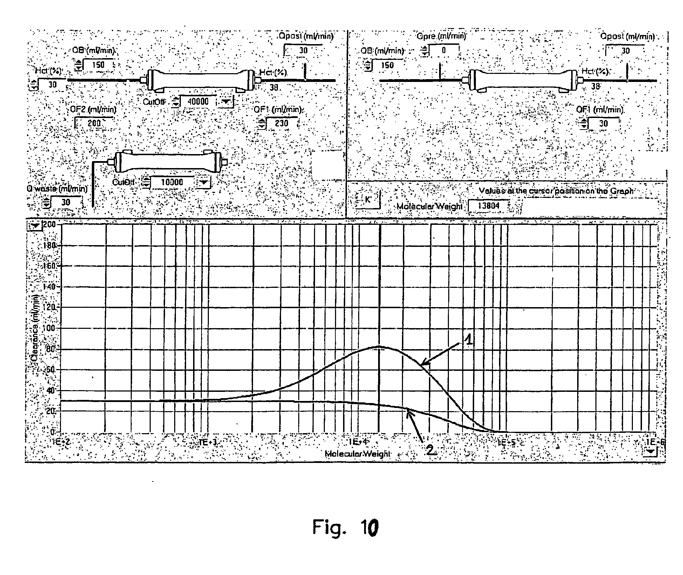

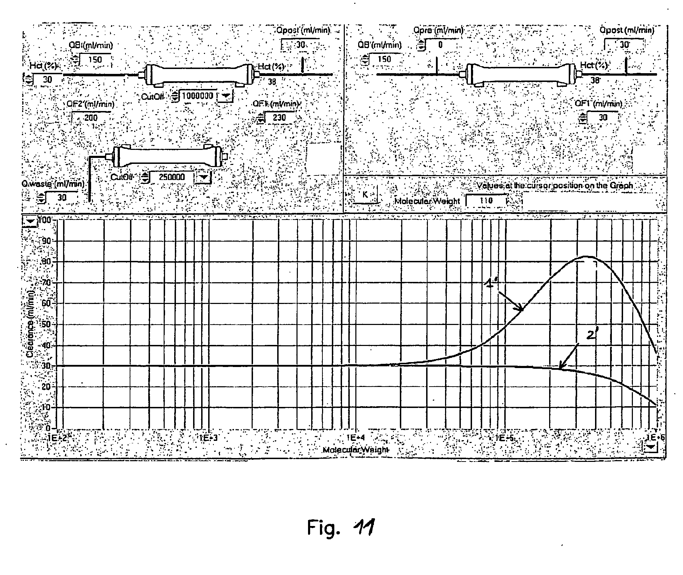

Des simulations ont été faites concernant des filtres à points de coupure différents. Les figures 10 et 11 représentent les résultats estimés en terme de clairance en fonction de la masse moléculaire des solutés pour deux configurations de dispositif selon l'invention. La figure 10 représente une première configuration ayant un échangeur de point de coupure égal à 40000 daltons, et une unité de traitement ayant un échangeur de point de coupure égal à 10000 daltons. La clairance (courbe 1) pour des molécules autour de 11000 daltons est très bonne alors que la clairance des petites molécules est gardée constante par rapport à un dispositif de fonctionnement ayant un unique filtre (courbe 2).

La figure 11 représente une deuxième configuration pour la plasmaphérèse ayant un échangeur de point de coupure égal à 1 000 000 daltons, et une unité de traitement ayant un échangeur de point de coupure égal à 250 000 daltons. La clairance (courbe 1') pour des molécules autour de 300 000 daltons est très bonne alors que la clairance des moyennes molécules est gardée constante par rapport à un dispositif de fonctionnement ayant un unique filtre (courbe 2').

L'invention apporte de nombreux avantages: elle permet de:
- multiplier par trois ou quatre, par rapport à un traitement standard de longue durée, l'épuration des molécules moyennes (ou grosses pour la plasmaphérèse) sans augmenter la quantité de liquide d'échange et sans changer l'épuration standard des petites molécules (petites et moyennes pour la plasmaphérèse),
- consommer beaucoup moins de liquide stérile, donc avoir un coût moins élevé,
éliminer suffisamment de molécules de taille moyenne,
garder les oligo-éléments et les nutriments qui sont réinjectés au patient,
filtrer à haut volume.

Particulièrement, dans la configuration illustrée en figure 5, de nombreux autres avantages sont présents. Un nombre minimal de moyens de réglage de débit est nécessité: une pompe péristaltique 101 sur la ligne artérielle et une pompe 103 sur la canalisation 40 permettent de faire fonctionner le dispositif.

Aussi, la position des moyens de réglage de débit est intelligemment étudiée : il n'est pas forcément besoin de pompe sur la canalisation 40, même si cela est envisageable, et le moyen de réglage de débit 103 n'a pas besoin d'être très puissant. Ceci permet d'avoir un fonctionnement en longue durée pour des soins intensifs en évitant une forte obturation des pores des différentes membranes.

Enfin, il a été pensé d'appliquer ce schéma de fonctionnement à un autre mode de traitement extracorporel de sang: la plasmaphérèse. Le fonctionnement plasmaphérèse atteint son optimum lorsque les membranes sont choisies et employées avec soin.

## Revendications

1. Dispositif de traitement extracorporel de sang comprenant:
- au moins un échangeur (1) comportant une membrane semi-perméable (6) divisant ledit échangeur en une première chambre (7) et une deuxième chambre (8), au moins une première entrée (2) pour le sang à traiter en communication de fluide avec la première chambre (7) de l'échangeur (1), une première sortie (4) de fluide en communication de fluide avec la première chambre (7) de l'échangeur (1) et une deuxième sortie (6) de fluide en communication de fluide avec la deuxième chambre (8) de l'échangeur (1),
- une ligne d'entrée (10) du sang à traiter raccordée à la première entrée (2) de l'échangeur (1),
- une ligne de sortie (11) du sang raccordée à la première sortie (4) de l'échangeur (1),
- au moins une unité de traitement (21) comportant une membrane semi-perméable (26) divisant l'unité de traitement (21) en une première chambre (27) et une deuxième chambre (28) et au moins une première entrée (22) de fluide en communication de fluide avec la deuxième chambre (28) de l'unité de traitement (21) et au moins une première sortie (24) de fluide en communication de fluide avec la première chambre (27) de l'unité de traitement (21).
- la deuxième sortie (5) de l'échangeur (1) étant en communication de fluide avec la première entrée (22) de l'unité de traitement (21),
- la première sortie (24) de l'unité de traitement (21) étant en communication de fluide avec la ligne d'entrée (10);
**caractérisé en ce que**:
- l'unité de traitement (21) comporte une deuxième sortie de fluide (25) en communication de fluide avec la deuxième chambre (28) de l'unité de traitement (21),
- la deuxième sortie (25) de l'unité de traitement (21) est en communication de fluide avec une première ligne de décharge (30) de liquide usé, ladite première ligne de décharge (30) pouvant relier la deuxième sortie (25) de l'unité de traitement (21) à un égout ou à un premier réservoir de liquide usé (31).

2. Dispositif selon la revendication précédente **caractérisé en ce que** la ligne d'entrée (10) du sang raccordée à la première entrée (2) de l'échangeur (1), la ligne de sortie (11) du sang raccordée à la première sortie (4) de l'échangeur et la première chambre (7) de l'échangeur font partie d'un circuit extracorporel de traitement de sang.

3. Dispositif selon la revendication 1 ou 2 où une première canalisation (12) est branchée entre la deuxième sortie (5) de l'échangeur (1) et la premier unité de traitement (21) pour effectuer la communication de fluide.

4. Dispositif selon une des revendications précédentes où une deuxième canalisation (40) est branchée entre la première sortie (24) de l'unité de traitement (21) et la première entrée (2) de l'échangeur (1) pour effectuer la communication de fluide.

5. Dispositif selon une quelconque des revendications précédentes **caractérisé en ce que** l'échangeur (1) comporte une deuxième entrée (3) en communication de fluide avec sa deuxième chambre (8) et en communication de fluide avec une première source de liquide de dialyse (9), le sang et le liquide de dialyse circulant dans chacune des deux chambres de l'échangeur en sens inverse.

6. Dispositif selon une quelconque des revendications précédentes en ce que la perméabilité aux molécules de la membrane (6) de l'échangeur (1) est supérieure à la perméabilité aux molécules de la membrane (26) de l'unité de traitement (21) au moins au-dessus d'une certaine masse moléculaire.

7. Dispositif selon une quelconque des revendications précédentes **caractérisé en ce que** la membrane (6) de l'échangeur (16) est une membrane à flux élevé et la membrane (26) de l'unité de traitement (21) est une membrane a flux faible.

8. Dispositif selon une quelconque des revendications 6 à 7 **caractérisé en ce que** le rapport du point de coupure de la première membrane (6) sur le point de coupure de la deuxième membrane (26) est inférieur ou égal a 3.

9. Dispositif selon une quelconque des revendications 6 à 8 **caractérisé en ce que** la différence de point de coupure entre La première membrane et la deuxième membrane est comprise entre 20000 dalton et 30000 dalton.

10. Dispositif selon une quelconque des revendications 6 à 9 **caractérisé en ce que** le point de coupure de la première membrane est inferieur ou égal à 40000 dalton.

11. Dispositif selon une quelconque des revendications 6 à 10 **caractérisé en ce que** le point de coupure de la deuxième membrane est inférieur ou égal à 10000 dalton.

12. Dispositif selon une quelconque des revendications précédentes **caractérisé en ce que** une ligne de post-dilution (50) est branchée sur la ligne de sortie (11) et est reliée à une première source de liquide stérile (51).

13. Dispositif selon une quelconque des revendications précédentes **caractérisé en ce qu'**il comprend une ligne de pré-dilution (60) en communication de fluide avec la ligne d'entrée (10) et reliée à une deuxième source de liquide stérile (61).

14. Dispositif une des revendications 1 à 13 **caractérisé en ce que** la ligne de pré-dilution (60) est branchée directement sur la deuxième canalisation (40).

15. Dispositif selon une des revendications 1 à 13 **caractérisé en ce que** la ligne de pré-dilution (60) est branchée directement sur la ligne d'entrée (10).

16. Dispositif selon une quelconque des revendications 1 à 8 ou 12 à 15 **caractérisé en ce que** l'échangeur est un plasma-filtre.

17. Dispositif selon la revendication 16 **caractérisé en ce que** le plasma-filtre a un point de coupure compris entre un million et cinq millions dalton.

18. Dispositif selon la revendication 16 ou 17 **caractérisé en ce que** l'unité de traitement comporte une membrane semi-perméable (26) ayant un point de coupure inférieur ou égal à 250000 dalton.

19. Dispositif selon une des revendications 16 à 18 **caractérisé en ce que** l'unité de traitement (21) comporte une membrane semi-perméable (26) ayant un point de coupure tel que sensiblement toutes les molécules d'albumine passent au travers de ladite membrane.

20. Dispositif selon une des revendications 16 à 19 **caractérisé en ce qu'**il comporte des moyens pour réagir sur au moins certaines molécules (70) actifs sur la première conduite (12) assurant la communication de fluide entre la deuxième sortie (5) de l'échangeur (1) et la première entrée (22) de l'unité de traitement (21).

21. Dispositif selon une quelconque des revendications précédentes comportant au moins:
- un échangeur auxiliaire (81) ayant une membrane (86) le séparant en une première chambre (87) en communication de fluide avec une première entrée (82) et une première sortie (84) et en une deuxième chambre (88) en communication de fluide avec au moins une deuxième sortie (85), et dont le point de coupure est inférieur aux points de coupure des membranes (6, 26) de l'échangeur et de l'unité de traitement,
- la première entrée (82) de l'échangeur auxiliaire (81) étant en communication de fluide avec la deuxième sortie (24) de l'unité de traitement (21),
- une des deux sorties (84, 85) de l'échangeur auxiliaire (81) étant en communication de fluide avec la première entrée (2) de l'échangeur (1).

22. Dispositif selon la revendication 21 **caractérisé en ce que** l'échangeur auxiliaire (81) comporte une deuxième entrée (83) en communication de fluide avec la deuxième chambre (88) de l'échangeur auxiliaire (81) et en communication de fluide avec une troisième source de liquide de dialyse (89),
- la première sortie (84) de l'échangeur auxiliaire (81) étant en communication de fluide avec la première entrée (82) de l'échangeur (1),
- la deuxième sortie (85) de l'échangeur auxiliaire (81) étant en communication de fluide avec un égout (91) par une deuxième ligne de décharge de liquide usé (90).

23. Dispositif selon une quelconque des revendications précédentes **caractérisé en ce qu'**il comporte des premiers moyens de réglage de débit (101) de liquide actifs sur la ligne d'entrée (10) reliée a la première entrée (2) de l'échangeur (1).

24. Dispositif selon la revendication 23 **caractérisé en ce que** les premiers moyens de réglage de débit (101) de liquide actifs sur la ligne d'entrée (10) sont exactement entre la première entrée (2) de l'échangeur (1) et le point de branchement (110) reliant la ligne d'entrée à la canalisation (40).

25. Dispositif selon la revendication 23 **caractérisé en ce que** les premiers moyens de réglage de débit (101) de liquide actifs sur la ligne d'entrée (10) sont en amont du point de branchement (110) reliant la ligne d'entrée (10) à la deuxième canalisation (40) et des deuxièmes moyens de réglage de débit (102) de liquide actifs sont sur la deuxième canalisation (40) reliant la première sortie (24) de l'unité de traitement (21) à la première entrée (2) de l'échangeur (1).

26. Dispositif selon une quelconque des revendications 23 à 25 **caractérisé en ce qu'**il comporte des troisièmes moyens de réglage de débit (103) de liquide actifs sur la conduite (12) reliant la deuxième sortie (5) de l'échangeur (1) à une des deux entrées (22, 23) de l'unité de traitement (21).

27. Dispositif selon une quelconque des revendications 23 à 26 **caractérisé en ce qu'**il comporte des quatrièmes moyens de réglage de débit (104) de liquide actifs sur la ligne de post-dilution (50).

28. Dispositif selon une quelconque des revendications 23 à 27 **caractérisé en ce qu'**il comporte des cinquièmes moyens de réglage de débit de liquide (105) actifs sur la ligne de décharge (30) de liquide usé reliant la deuxième sortie (25) de l'unité de traitement (21) à un égout (31).

29. Dispositif selon une quelconque des revendications 23 à 28 **caractérisé en ce qu'**il comporte des sixièmes moyens de réglage de débit de liquide (106) actifs sur la ligne de pré-dilution (60).

30. Dispositif selon une quelconque des revendications 27 à 29 **caractérisé en ce** la première source (51) de liquide stérile pour la post-dilution est une poche de liquide stérile et en ce que le premier réservoir (31) de liquide usé relié à la ligne de décharge en sortie de l'unité de traitement est une poche de liquide usé.

31. Dispositif selon la revendication 30 **caractérise en ce qu'**il comporte une première balance (120) pour mesurer le poids de la poche de liquide stérile (51) et une deuxième balance (121) pour mesurer le poids de la poche de liquide usé (31).

32. Dispositif selon la revendication 30 **caractérisé en ce qu'**il comporte une unique balance (120, 121) pour mesurer le poids total de la poche de liquide stérile (51) et de la poche de liquide usé (31).

33. Dispositif selon la revendication 31 ou 32 **caractérisé en ce qu'**il comporte une unité de calcul et de commande (130) pour recevoir les signaux émis par au moins une balance (120, 121) et pour contrôler les moyens de réglage de débit de liquide (101, 102, 103, 104, 105).

## Claims

1. Extracorporeal blood treatment device comprising:
- at least one exchanger (1) having a semipermeable membrane (6) dividing said exchanger into a first compartment (7) and a second compartment (8), at least a first inlet (2) for blood to be treated in fluid communication with the first compartment (7) of the exchanger (1), a first fluid outlet (4) in fluid communication with the first compartment (7) of the exchanger (1), and a second fluid outlet (5) in fluid communication with the second compartment (8) of the exchanger (1),
- an inlet line (10) for blood to be treated connected to the first inlet (2) of the exchanger (1),
- an outlet line (11) for blood connected to the first outlet (4) of the exchanger (1),
- at least one treatment unit (21) having a semipermeable membrane (26) dividing the treatment unit (21) into a first compartment (27) and a second compartment (28), and at least a first fluid inlet (22) in fluid communication with the second compartment (28) of the treatment unit (21), and at least a first fluid outlet (24) in fluid communication with the first compartment (27) of the treatment unit (21),
- the second outlet (5) of the exchanger (1) being in fluid communication with the first inlet (22) of the treatment unit (21),
- the first outlet (24) of the treatment unit (21) is in fluid communication with the inlet line (10);
**characterized in that**:
- the treatment unit (21) comprises a second fluid outlet (25) in fluid communication with the second compartment (28) of the treatment unit (21),
- the second outlet (25) of the treatment unit (21) is in fluid communication with a first discharge line (30) for used liquid, said first discharge line (30) being able to connect the second outlet (25) of the treatment unit (21) with a drain or a first tank for used liquid (31).

2. Device according to the preceding claim, **characterized in that** the inlet line (10) for blood connected to the first inlet (2) of the exchanger (1), the outlet line (11) for blood connected to the first outlet (4) of the exchanger and the first compartment (7) of the exchanger are part of an extracorporeal blood treatment circuit.

3. Device according to claim 1 or 2, wherein a first channel is coupled between the second outlet (5) of the exchanger (1) and the treatment unit (21) for carrying out fluid communication.

4. Device according to one of the preceding claims, wherein a second conduit (40) is coupled between the first outlet (24) of the treatment unit (21) and the first inlet (2) of the exchanger (1) for carrying out fluid communication.

5. Device according to any one of the preceding claims, **characterized in that** the exchanger (1) comprises a second inlet (3) in common with its second compartment (8) and in fluid communication with a first dialysis liquid source (9), blood and dialysis liquid circulating in each one of the two compartments of the exchanger in opposite directions.

6. Device according to any one of the preceding claims, **characterized in that** the permeability to molecules of the membrane (6) of the exchanger (1) is higher than the permeability to molecules of the membrane (26) of the treatment unit (21) at least above a given molecular mass.

7. Device according to any one of the preceding claims, **characterized in that** the membrane (6) of the exchanger (1) is a high flow membrane and the membrane (26) of the treatment unit (21) is a low flow membrane.

8. Device according to any one of the claims 6 to 7, **characterized in that** the ratio of the cut-off point of the first membrane (6) to the cut-off point of the second membrane (26) is lower than or equal to 3.

9. Device according to any one of the claims 6, to 8, **characterized in that** the cut-off point difference between the first membrane and the second membrane is of 20.000 to 30.000 Dalton.

10. Device according to any one of the claims 6 to 9, **characterized in that** the cut-off point of the first membrane is lower than or equal to 40.000 Dalton.

11. Device according to any one of the claims 6 to 10, **characterized in that** the cut-off point of the second membrane is lower than or equal to 10.000 Dalton.

12. Device according to any one of the preceding claims, **characterized in that** a post-dilution line (50) is coupled onto the outlet line (11) and is connected to a first sterile liquid source (51).

13. Device according to any one of the preceding claims, **characterized in that** it comprises a pre-dilution line (60) in fluid communication with the inlet line (10) and connected to a second sterile liquid source (61).

14. Device according to one of the claims 1 to 13, **characterized in that** the pre-dilution line (60) is coupled directly onto the second canalization (40).

15. Device according to any one of the claims 1 to 13, **characterized in that** the pre-dilution line (60) is coupled directly onto the inlet line (10).

16. Device according to any one of the claims 1 to 8 or 12 to 15, **characterized in that** the exchanger is a plasma filter.

17. Device according to claim 16, **characterized in that** the plasma filter has a cut-off point of one million to five million Dalton.

18. Device according to claim 16 or 17, **characterized in that** the treatment unit comprises a semipermeable membrane (26) with a cut-off point lower than or equal to 250.000 Dalton.

19. Device according to one of the claims 16 to 18, **characterized in that** the treatment unit (21) comprises a semipermeable membrane (26) having such a cut-off point that substantially all albumin molecules get through said membrane.

20. Device according to one of the claims 16 to 19, **characterized in that** it comprises means for reacting to at least some molecules (70) acting upon the first conduit (12) assuring the fluid communication between the second outlet (5) of the exchanger (1) and the first inlet (22) of the treatment unit (21).

21. Device according to any one of the preceding claims, comprising at least:
- one auxiliary exchanger (81) having a membrane separating it into a first compartment (87) in fluid communication with a first inlet (82) and a first outlet (84), and into a second compartment (88) in fluid communication with at least one second outlet (85), and whose cut-off point is lower than the cut-off points of the membranes (6, 26) of the exchanger and of the treatment unit,
- the first inlet (82) of the auxiliary exchanger (81) being in fluid communication with the second outlet (24) of the treatment unit (21),
- one of the two outlets (84, 85) of the auxiliary exchanger (81) being in fluid communication with the first inlet (2) of the exchanger (1).

22. Device according to claim 21, **characterized in that** the auxiliary exchanger (81) comprises a second inlet (83) in fluid communication with the second compartment (88) of the auxiliary exchanger (81) and in fluid communication with a third dialysis liquid source (89),
- the first outlet (84) of the auxiliary exchanger (81) being in fluid communication with the first inlet (2) of the exchanger (1),
- the second outlet (85) of the auxiliary exchanger (81) being in fluid communication with a drain (91) through a second discharge line for used liquid (90). ,

23. Device according to any one of the preceding claims, **characterized in that** it comprises first liquid flow rate adjusting means (101) acting upon the inlet line (10) connected to the first inlet (2) of the exchanger (1).

24. Device according to claim 23, **characterized in that** the first liquid flow rate adjusting means (101) acting upon the inlet line (10) lie exactly between the first inlet (2) of the exchanger (1) and the coupling point (110) connecting the inlet line to the canalization (40).

25. Device according to claim 23, **characterized in that** the first liquid flow rate adjusting means (101) acting upon the inlet line (10) lie upstream from the coupling point (110) connecting the inlet line (10) to the second canalization (40), and second active liquid flow rate adjusting means (102) lie on the second canalization (40) connecting the first outlet (24) of the treatment unit (21) to the first inlet (2) of the exchanger (1).

26. Device according to any one of the claims 23 to 25, **characterized in that** it comprises third liquid flow rate adjusting means (103) acting upon the conduit (12) connecting the second outlet (5) of the exchanger (1) to one of the two inlets (22, 23) of the treatment unit (21).

27. Device according to any one of the claims 23 to 26, **characterized in that** it comprises fourth liquid.flow rate adjusting means (104) acting upon the post-dilution line (50).

28. Device according to any one of the claims 23 to 27, **characterized in that** it comprises fifth liquid flow rate adjusting means (105) acting upon the discharge line (30) for used liquid connecting the second outlet (25) of the treatment unit (21) to a drain (31).

29. Device according to any one of the claims 23 to 28, **characterized in that** it comprises sixth liquid flow rate adjusting means (106) acting upon the pre-dilution line (60).

30. Device according to any one of the claims 27 to 29, **characterized in that** the first sterile liquid source (51) for post-dilution is a sterile liquid bag, and **in that** the first tank (31) for used liquid connected to the discharge line getting out of the treatment unit is a used liquid bag.

31. Device according to claim 30, **characterized in that** it comprises a first set of scales (120) for measuring the weight of the sterile liquid bag (51) and a second set of scales (121) for measuring the weight of the used liquid bag (31).

32. Device according to claim 30, **characterized in that** it comprises one set of scales (120, 121) for measuring the total weight of the sterile liquid bag (51) and of the used liquid bag (31).

33. Device according to claim 31 or 32, **characterized in that** it comprises a computing and control unit (130) for receiving the signals sent by at least one set of scales (120, 121) and for controlling the liquid flow rate adjusting means (101, 102, 103, 104, 105).

## Patentansprüche

1. Extrakorporale Blutbehandlungsvorrichtung umfassend:
- mindestens einen Austauscher (1) enthaltend eine semipermeable Membran (6), die den benannten Austauscher in einen ersten Abteil (7) und einen zweiten Abteil (8) trennt, mindestens einen ersten Eingang (2) für das zu behandelnde Blut, der mit dem ersten Abteil (7) des Austauschers (1) in Fluidkommunikation steht, einen ersten Fluidausgang (4), der mit dem ersten Abteil (7) des Austauschers (1) in Fluidkommunikation steht, und einen zweiten Fluidausgang (5), der mit dem zweiten Abteil (8) des Austauschers (1) in Fluidkommunikation steht,
- eine Eingangsleitung (10) für das zu behandelnde Blut, die mit dem ersten Eingang (2) des Austauschers (1) verbunden ist;
- eine Ausgangsleitung (11) für das Blut, die mit dem ersten Ausgang (4) des Austauschers (1) verbunden ist;
- mindestens eine Behandlungseinheit (21) enthaltend eine semipermeable Membran (26), die die Behandlungseinheit (21) in einen ersten Abteil (27) und einen zweiten Abteil (28) trennt, und mindestens einen ersten Fluideingang (22), der mit dem zweiten Abteil (28) der Behandlungseinheit (21) in Fluidkommunikation steht, und mindestens einen ersten Fluidausgang (24), der mit dem ersten Abteil (27) der Behandlungseinheit (21) in Fluidkommunikation steht,
- wobei der zweite Ausgang (5) des Austauschers (1) mit dem ersten Eingang (22) der Behandlungseinheit (21) in Fluidkommunikation steht,
- wobei der erste Eingang (24) der Behandlungseinheit (21) mit der Eingangsleitung (10) in Fluidkommunikation steht,
**dadurch gekennzeichnet, daß**:
- die Behandlungseinheit (21) einen zweiten Fluidausgang (25) umfasst, der mit dem zweiten Abteil (28) der Behandlungseinheit (21) in Fluidkommunikation steht,
- der zweite Ausgang (25) der Behandlungseinheit (21) mit einer ersten Auslaufleitung (30) für die gebrauchte Flüssigkeit in Fluidkommunikation steht, wobei die benannte erste Auslaufleitung (30) den zweiten Ausgang (25) der Behandlungseinheit (21) mit einem Abflußkanal oder mit einem ersten Behälter für gebrauchte Flüssigkeit (31) verbinden kann.

2. Vorrichtung nach dem vorherigen Anspruch, **dadurch gekennzeichnet, daß** die Eingangsleitung (10) für das Blut, die mit dem ersten Eingang (2) des Austauschers (1) verbunden ist, die Ausgangsleitung (11) für das Blut, die mit dem ersten Ausgang (4) des Austauschers verbunden ist, und der erste Abteil (7) des Austauschers Teile eines extrakorporalen Blutbehandlungskreislaufs sind.

3. Vorrichtung nach Anspruch 1 oder 2, worin eine erste Kanalisation zwischen dem zweiten Ausgang (5) des Austauschers (1) und dem ersten der Behandlungseinheit (21) angeschlossen ist, um die Fluidkommunikation durchzuführen.

4. Vorrichtung nach einem der vorherigen Ansprüche, worin eine zweite Kanalisation (40) zwischen dem ersten Ausgang (24) der Behandlungseinheit (21) und dem ersten Eingang (2) des Austauschers (1) angeschlossen ist, um die Fluidkommunikation durchzuführen.

5. Vorrichtung nach irgendeinem der vorherigen Ansprüche, **dadurch gekennzeichnet, daß** der Austauscher (1) einen zweiten Eingang (3) umfasst, der gemeinsam mit dessen zweiten Abteil (8) ist und mit einer ersten Dialyseflüssigkeitsquelle (9) in Fluidkommunikation steht, wobei das Blut und die Dialyseflüssigkeit in jedem der beiden Abteile des Austauschers in umgekehrter Richtung umlaufen.

6. Vorrichtung nach irgendeinem der vorherigen Ansprüche, **dadurch gekennzeichnet, daß** die Molekülpermeabilität der Membran (6) des Austauschers (1) höher ist als die Molekülpermeabilität der Membran (26) der Behandlungseinheit (21) mindestens oberhalb einer bestimmten Molekülmasse.

7. Vorrichtung nach irgendeinem der vorherigen Ansprüche, **dadurch gekennzeichnet, daß** die Membran (6) des Austauschers (1) eine Hochflussmembran ist und die Membran (26) der Behandlungseinheit (21) eine Niederflussmembran ist.

8. Vorrichtung nach irgendeinem der Ansprüche 6 bis 7, **dadurch gekennzeichnet, daß** das Verhältnis des Grenzpunkts der ersten Membran (6) zum Grenzpunkt der zweiten Membran (26) 3 oder weniger beträgt.

9. Vorrichtung nach irgendeinem der Ansprüche 6 bis 8, **dadurch gekennzeichnet, daß** der Grenzpunktunterschied zwischen der ersten Membran und der zweiten Membran 20.000 Dalton bis 30.000 Dalton beträgt.

10. Vorrichtung nach irgendeinem der Ansprüche 6 bis 9, **dadurch gekennzeichnet, daß** der Grenzpunkt der ersten Membran 40.000 Dalton oder weniger beträgt.

11. Vorrichtung nach irgendeinem der Ansprüche 6 bis 10, **dadurch gekennzeichnet, daß** der Grenzpunkt der zweiten Membran 10.000 oder weniger beträgt.

12. Vorrichtung nach irgendeinem der vorherigen Ansprüche, **dadurch gekennzeichnet, daß** eine Nachverdünnungsleitung (50) an die Ausgangsleitung (11) angeschlossen ist und mit einer ersten Quelle von steriler Flüssigkeit (51) verbunden ist.

13. Vorrichtung nach irgendeinem der vorherigen Ansprüche, **dadurch gekennzeichnet, daß** sie eine Vorverdünnungsleitung (60) umfasst, die mit der Eingangsleitung (10) in Fluidkommunikation steht und mit einer zweiten Quelle von steriler Flüssigkeit (61) verbunden ist.

14. Vorrichtung nach einem der Ansprüche 1 bis 13, **dadurch gekennzeichnet, daß** die Vorverdünnungsleitung (60) unmittelbar an die zweite Kanalisation (40) angeschlossen ist.

15. Vorrichtung nach einem der Ansprüche 1 bis 13, **dadurch gekennzeichnet, daß** die Vorverdünnungsleitung (60) unmittelbar an die Eingangsleitung (10) angeschlossen ist.

16. Vorrichtung nach irgendeinem der Ansprüche 1 bis 8 oder 12 bis 15, **dadurch gekennzeichnet, daß** der Austauscher ein Plasmafilter ist.

17. Vorrichtung nach Anspruch 16, **dadurch gekennzeichnet, daß** der Plasmafilter einen Grenzpunkt von 1.000.000 bis 5.000.000 Dalton besitzt.

18. Vorrichtung nach Anspruch 16 oder 17, **dadurch gekennzeichnet, daß** die Behandlungseinheit eine semipermeable Membran (26) mit einem Grenzpunkt von 250.000 Dalton oder weniger besitzt.

19. Vorrichtung nach einem der Ansprüche 16 bis 18, **dadurch gekennzeichnet, daß** die Behandlungseinheit (21) eine semipermeable Membran (26) mit einem solchen Grenzpunkt besitzt, daß wesentlich alle Albuminmoleküle durch die benannte Membran durchgehen.

20. Vorrichtung nach einem der Ansprüche 16 bis 19, **dadurch gekennzeichnet, daß** sie Mittel zur Reaktion gegen mindestens bestimmte Moleküle (70) aufweist, die auf die erste Rohrleitung (12) wirken, die die Fluidkommunikation zwischen dem zweiten Ausgang (5) des Austauschers (1) und dem ersten Eingang (22) der Behandlungseinheit (21) gewährleistet.

21. Vorrichtung nach irgendeinem der vorherigen Ansprüche, umfassend mindestens:
- einen Nebenaustauscher (81) enthaltend eine Membran (86), die ihn in einen ersten, mit einem ersten Eingang (82) und einem ersten Ausgang (84) in Fluidkommunikation stehenden Abteil (87) und in einen zweiten, mit mindestens einem zweiten Ausgang (85) in Fluidkommunikation stehenden Abteil (88) trennt, und deren Grenzpunkt kleiner ist als die Grenzpunkte der Membranen (6, 26) des Austauschers und der Behandlungseinheit,
- wobei der erste Eingang (82) des Nebenaustauschers (81) mit dem zweiten Ausgang (24) der Behandlungseinheit (21) in Fluidkommunikation steht,
- wobei einer der beiden Ausgänge (84, 85) des Nebenaustauschers (81) mit dem ersten Eingang (2) des Austauschers (1) in Fluidkommunikation steht.

22. Vorrichtung nach Anspruch 21, **dadurch gekennzeichnet, daß** der Nebenaustauscher (81) einen zweiten Eingang (83) umfasst, der mit dem zweiten Abteil (88) des Nebenaustauschers (81) und mit einer dritten Quelle von Dialyseflüssigkeit (89) in Fluidkommunikation steht,
- wobei der erste Ausgang (84) des Nebenaustauschers (81) mit dem ersten Eingang (2) des Austauschers (1) in Fluidkommunikation steht,
- wobei der zweite Ausgang (85) des Nebenaustauschers (81) mit einem Abflußkanal (91) durch eine zweite Auslaufleitung für gebrauchte Flüssigkeit (90) in Fluidkommunikation steht.

23. Vorrichtung nach irgendeinem der vorherigen Ansprüche, **dadurch gekennzeichnet, daß** sie erste Flüssigkeitsfördermengeregelmittel (101) umfasst, die auf die mit dem ersten Eingang (2) des Austauschers (1) verbundene Eingangsleitung (10) wirken.

24. Vorrichtung nach Anspruch 23, **dadurch gekennzeichnet, daß** die ersten, auf die Eingangsleitung (10) wirkenden Flüssigkeitsfördermengeregelmittel (101) zwischen dem ersten Eingang (2) des Austauschers (1) und dem die Eingangsleitung mit der Kanalisation (40) verbindenden Anschlußpunkt (110) genau liegen.

25. Vorrichtung nach Anspruch 23, **dadurch gekennzeichnet, daß** die ersten Flüssigkeitsfördermengeregelmittel (101), die auf die Eingangsleitung (10) wirken, aufwärts vom die Eingangsleitung (10) mit der zweiten Kanalisation (40) verbindenden Anschlußpunkt liegen, und zweite Flüssigkeitsfördermengeregelmittel (102) auf die zweite, den ersten Ausgang (24) der Behandlungseinheit (21) mit dem ersten Eingang (2) des Austauschers (1) verbindende Kanalisation (40) wirken.

26. Vorrichtung nach irgendeinem der Ansprüche 23 bis 25, **dadurch gekennzeichnet, daß** sie dritte Flüssigkeitsfördermengeregelmittel (103), die auf die den zweiten Ausgang (5) des Austauschers (1) mit einem der beiden Eingänge (22, 23) der Behandlungseinheit (21) verbindende Rohrleitung (12) wirken, umfasst.

27. Vorrichtung nach irgendeinem der Ansprüche 23 bis 26, **dadurch gekennzeichnet, daß** sie vierte Flüssigkeitsfördermengeregelmittel (104), die auf die Nachverdünnungsleitung (50) wirken, umfasst.

28. Vorrichtung nach irgendeinem der Ansprüche 23 bis 27, **dadurch gekennzeichnet, daß** sie fünfte Flüssigkeitsfördermengeregelmittel (105), die auf die den zweiten Ausgang (25) der Behandlungseinheit (21) mit einem Abflußkanal (31) verbindende Auslaufleitung für gebrauchte Flüssigkeit (30) wirken, umfasst.

29. Vorrichtung nach irgendeinem der Ansprüche 23 bis 28, **dadurch gekennzeichnet, daß** sie sechste Flüssigkeitsfördermengeregelmittel (106), die auf die Vorverdünnungsleitung (60) wirken, umfasst.

30. Vorrichtung nach irgendeinem der Ansprüche 27 bis 29, **dadurch gekennzeichnet, daß** die erste Quelle von steriler Flüssigkeit (51) für die Nachverdünnung ein steriler Flüssigkeitsbeutel ist, und daß der erste, mit der Auslaufleitung ausgehend von der Behandlungseinheit verbundene Behälter von gebrauchter Flüssigkeit (31) ein gebrauchter Flüssigkeitsbeutel ist.

31. Vorrichtung nach Anspruch 30, **dadurch gekennzeichnet, daß** sie eine erste Waage (120) zur Messung des Gewichts des sterilen Flüssigkeitsbeutels (51) und eine zweite Waage (121) zur Messung des Gewichts des gebrauchtes Flüssigkeitsbeutels (31) umfasst.

32. Vorrichtung nach Anspruch 30, **dadurch gekennzeichnet, daß** sie eine einzige Waage (120, 121) zur Messung des Gesamtgewichts des sterilen Flüssigkeitsbeutels (51) und des gebrauchten Flüssigkeitsbeutels (31) umfasst.

33. Vorrichtung nach Anspruch 31 oder 32, **dadurch gekennzeichnet, daß** sie eine Rechen- und Steuerungseinheit (130) umfasst, um die von mindestens einer Waage (120, 121) ausgegebenen Signale zu empfangen und die Flüssigkeitsfördermengeregelmittel (101, 102, 103, 104, 105) zu steuern.
